# EUROPEAN PATENT SPECIFICATION

(11) **EP 1 516 919 B1**
(45) Date of publication and mention of the grant of the patent: **11.04.2007**
(21) Application number: 04253377.8
(22) Date of filing: 07.06.2004
(51) Int. Cl.: C12M 1/02, B01F 7/30, B01F 11/00, C12M 1/24

(54) **Cell culture vessel for the automated processing of cell cultures**
Zellkulturgefäss für die automatische Verarbeitung von Zellkulturen
Récipient de culture de cellules pour la culture automatisée de cultures de cellules

(30) Priority: 19.09.2003 EP 03255896
(43) Date of publication of application: 23.03.2005
(73) Proprietor: The Automation Partnership (Cambridge) Limited, Royston, Hertfordshire SG8 5WY (GB)
(72) Inventor: Bargh, Adrian Neil, London N8 7LS (GB)
(74) Representative: Brunner, Michael John

(56) References cited:
- EP-A- 0 747 476
- US-A- 3 854 704
- US-A- 3 913 895
- US-A- 3 955 802
- US-A- 4 204 774

## Description

The present invention relates to a cell culture aeration unit and in particular to a cell culture aeration assembly and method for aerating cell culture media. The cell culture aeration assembly is suitable for use in the production of cell culture products and in particular to the automated production of protein.

The production of specific proteins from cloned genes are essential first steps in many areas of research and development in the pharmaceutical industry. Generally, the protein of interest (or target protein) is produced within, or secreted by, cultured cells or host organisms and the target protein is recovered from the culture fluid or the cells themselves. More specifically, the gene for the target protein is linked to the appropriate DNA elements controlling transcription and translation in the host organism or cells using standard recombinant DNA techniques. During the growth of the recombinant organism or cells in the correct physical and chemical environment to trigger transcription and translation of the cloned gene, the target protein is produced. Typical host organisms and cell types that might be used in this process include bacteria such as *E.coli,* yeast and insect cells.

A common problem experienced with this method of producing protein is that the genes required for the production of the target proteins are not generally native to the host organism or cells used. Not only are the genes from a different species to the host organism or cells, the target proteins are often only found in certain specialised cell types. A result of this is that the host organism or cells used may comprise a non-optimal environment for the production, stability, and proper folding of the target protein.

Extensive efforts must thus be made to find appropriate culture conditions for individual strains of the host cell types used and to address nuances of modification of the gene structure in order to facilitate the production of sufficient amounts of the target protein in the desired form. A further problem experienced is that variation in the dynamics of the expression of the target protein, i.e. the rate of production and the point in the growth cycle at which expression is initiated, can have a major impact on the quality and quantity of target protein produced. To identify appropriate conditions requires the evaluation of hundreds and sometimes thousands of combinations of variables.

Methods to identify appropriate conditions for protein production are currently carried out manually or in a semi-automated fashion. Such methods, however, are slow and involve challenging experimental schedules including frequent growth monitoring which, of course, is difficult to marry with normal working practices.

Furthermore, there is a limitation to the number of experiments that can be carried out in parallel and variations in operational procedures often occur creating inconsistent and non-reproducible results. An impact of the labile nature of the desired target proteins is that such variations may substantially affect the quality of the final product. There is also a health risk to staff when carrying out such large numbers of experiments such as RSI, fatigue, and exposure to genetically modified organisms, for example.

Process steps in the production of protein which have to date made it difficult to carry on its production in a fully automated fashion include measurement of optical density of the bacterial cultures.

Conventionally, culture media are aerated by mechanically shaking the culture vessel or, where higher levels of oxygenation of the culture are required, aeration is achieved by stirring the culture medium with an impeller. The impeller is usually mounted on a rod, inserted into the vessel, and rotated about the axis of the rod. The impeller may be driven directly through connection to a motor, or indirectly from outside the culture vessel using a magnetic drive.

Alternatively, it is known to introduce a hollow rod into a culture vessel and then aerate the culture medium by passing air through the rod into the culture vessel. As the air exits the lower end of the rod it breaks into bubbles which aerate the medium. This technique is known as "sparging."

Where multiple cultures are required at low volume and in parallel, the standard aeration technique used is shaking as the manufacture of parallel impellers or sparging rods and their respective drive systems at small scale is complex and expensive. Shaking of cultures in vessels of small volume does not however generally provide the levels of aeration required to satisfy the oxygen requirements of high density cultures of bacterial cells such as *E.coli .* There is currently no pragmatic and reliable solution for the effective aeration of cultures of E.coli and other cell types such as insect cells at volumes of 10mls or less in a space efficient parallel fashion, for example, where more than 6 cultures in parallel are aerated.

Generally, culture vessels designed to provide the levels of oxygenation required to support the aerobic growth of organisms and cell types such as *E.coli* and insect cells have a structure that requires the transfer of the culture to a separate vessel for the harvesting of the cells by centrifugation. Avoiding such a requirement for the transfer of the culture would allow for improved automated manipulation of the culture vessel or vessels.

It is an object of the present invention to provide a cell culture aeration unit and cell culture aeration assembly suitable for automated operation which will enable aeration of cultures in parallel, and help maintain the cells in suspension and prevent them from settling to the bottom of the vessel.

The present invention will allow for an automated system which will enable the optimisation of culture conditions and dynamics, host strains and genetic modifications in order to produce and purify proteins of the appropriate quality and quantity.

According to a first aspect of the present invention, there is provided a system for aerating a plurality of cell samples contained in a plurality of wells, the system comprising:
a plurality of wells arranged in a culture block;
at least one stirring rod for each of the plurality of wells;
first and second platens arranged above the culture block and provided with connecting means for coupling each platen to each rod;
wherein relative motion between the first and second platens (parallel to one another) results in the rods stirring the contents of the wells.

Typically, the stirring rod extends substantially the length of the culture vessel so that the first end of the stirring rod lies near the closed end of the vessel. In this way, the stirring rod helps to maintain the cells in the culture in suspension which would otherwise settle to the bottom of the culture vessel.

Preferably, the stirring rod moves about a pivot point intermediate the first and second ends of the stirring rod and adjacent the open end of the culture vessel. The stirring rod may be moved in a rotating motion about the pivot point. Rotation of the stirring rod about the pivot point is such that it transcribes a cone shape, the pivot point corresponding to the apex of the cone.

Preferably, the vessel is made of a transparent material, for example, polycarbonate.

The stirring rod may be made of a stainless steel or a Nickel Titanium alloy.

Optionally, the culture aeration unit may include a second stirring rod introduced into each of the wells. The stirring rods may be of equal length or of different lengths. Preferably, the second stirring rod extends approximately half the length of the vessel so that upper and lower portions of the culture are generally mixed and aerated equally by the first and second stirring rods respectively. This arrangement generally allows for a more uniform stirring and aeration of the culture.

The culture aeration assembly may include a seal sandwiched between the lid and the open end of the culture vessel block, the seal having a plurality of perforations to accommodate access of the stirring rods into a corresponding well.

Preferably, the lid has an upper and lower portion, the upper portion of the lid being movable relative to the lower portion of the lid which is stationary relative to the culture vessel block.

The second end of each stirring rod extend upwardly from the lower lid portion to engage the upper lid portion. Means for moving the lid may comprise a cam in communication with the upper lid portion. Movement of the upper lid portion relative to the lower lid portion moves each stirring rod so that it pivots about a point above the surface of the culture medium.

There is a seal on the lower platen that may be either a single mat affixed to the lower surface of the lower platen with openings to allow the rods to protrude into the culture vessel block or it may consist of a series of individual seals, in the form of "O"-rings within the lower platen. The flexible nature of the seal allows the stirring rods to move while also maintaining the integrity of the seal. The seal which is resistant to the passage of both air and liquid, helps limit contamination of the wells from extemal of the cell culture block and cross-contamination from one well to another.

The air is delivered to the cell culture vessels through air inlets in the lid of the cell culture block.

The invention will be more clearly understood by way of description of an embodiment thereof given by way of example only with reference to the accompanying drawings in which:-
Fig. 1 shows a plan view of an automated system for manipulating the culture aeration assembly and culture vessel assembly according to the present invention in the production of protein;

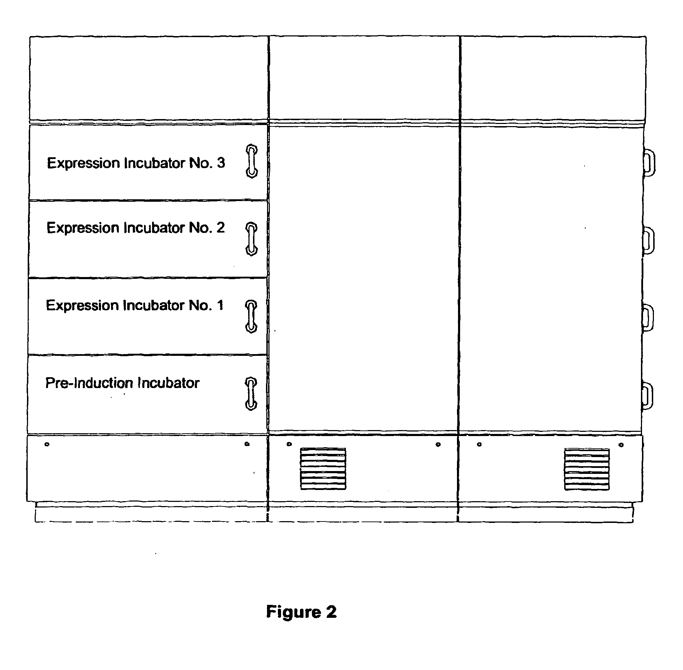

Fig. 2 is an end view in the direction of the arrow A of the automated system of fig. 1 showing an arrangement of incubators;
Fig. 3 is an end view of the automated system of fig. 1 in the direction of the arrow B showing manual access ports to the automated system;
Fig. 4 is a perspective view and from above of an embodiment of culture aeration assembly (shown here without the upper lid portion) and culture vessel assembly according to the present invention;
Fig. 5 is a cross-sectional side view of an alternative embodiment of the culture aeration assembly and culture vessel assembly clearly showing the upper lid portion of the culture aeration assembly ;
Fig. 6 is a side view of the culture aeration assembly of fig.5 shown here without the culture vessel assembly;
Fig. 7 is a perspective view and from below of the culture aeration unit of fig. 6 shown here without securing arms;
Fig. 8 is the perspective view and from the side of the culture vessel assembly of fig. 5 shown here without the culture aeration assembly;
Fig. 9 is an end view in the direction of the arrow F of the culture vessel assembly of Fig. 8.

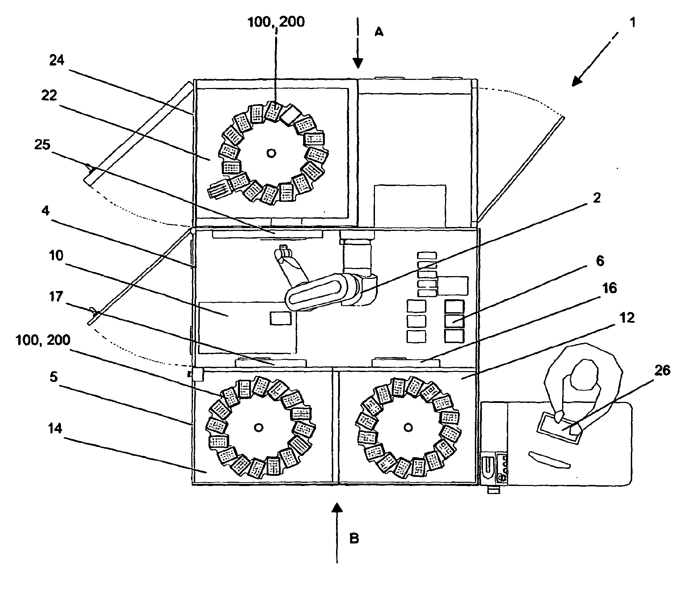

Referring to the drawings and initially to Fig. 1, there is shown an automated cell culture system generally indicated by the reference numeral 1 for producing a protein, the system comprising an anthropomorphic robot 2 operating within a temperature and air controlled enclosure 4 located centrally of the system 1. Within the central enclosure 4 there is also located, a liquid handling unit 6 for dispensing liquid media (e.g. culture media and antibiotics) at various steps of the process for synthesising the protein, and a centrifuge 10 for cell harvest and lysate clarification.

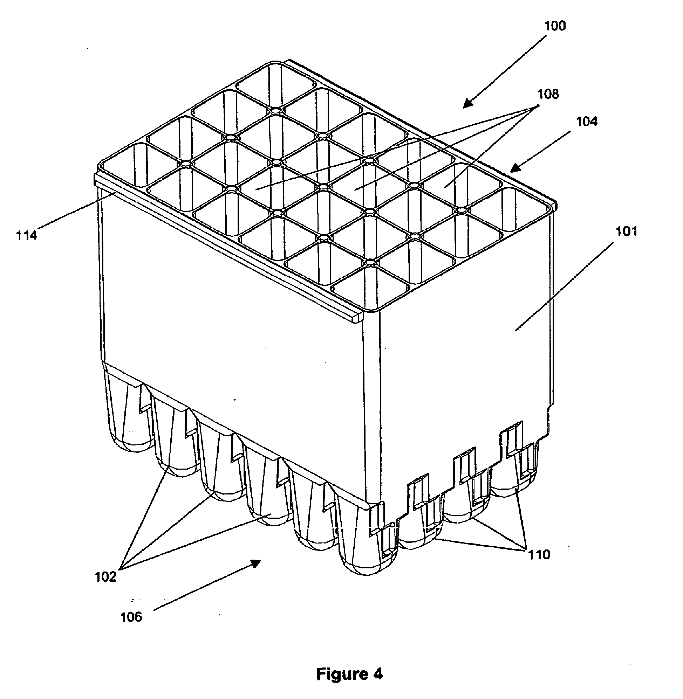

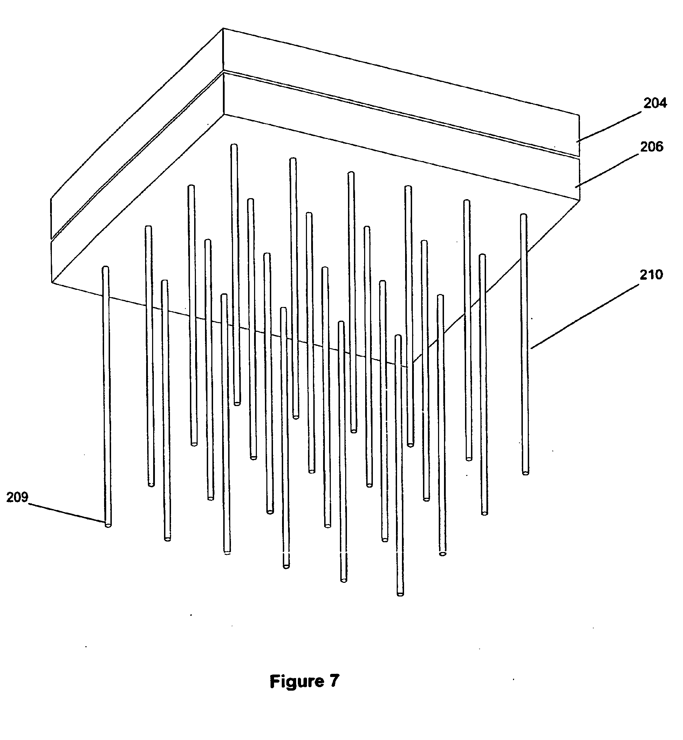

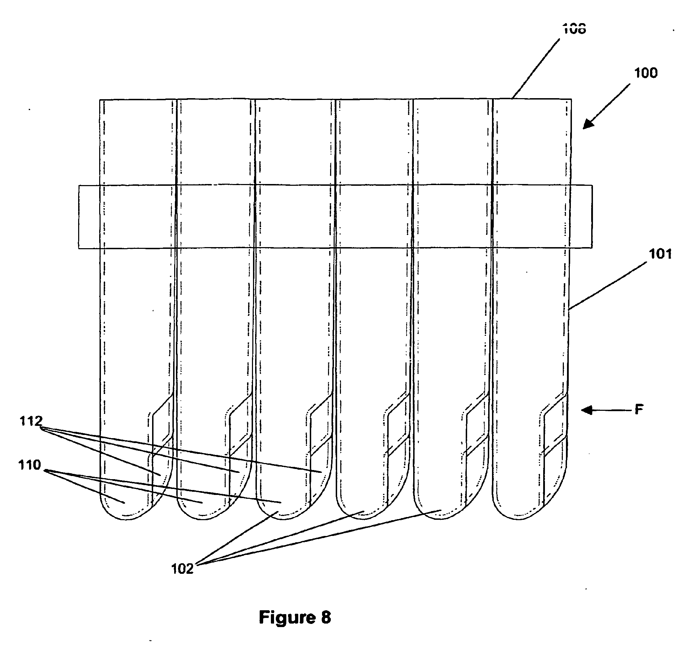

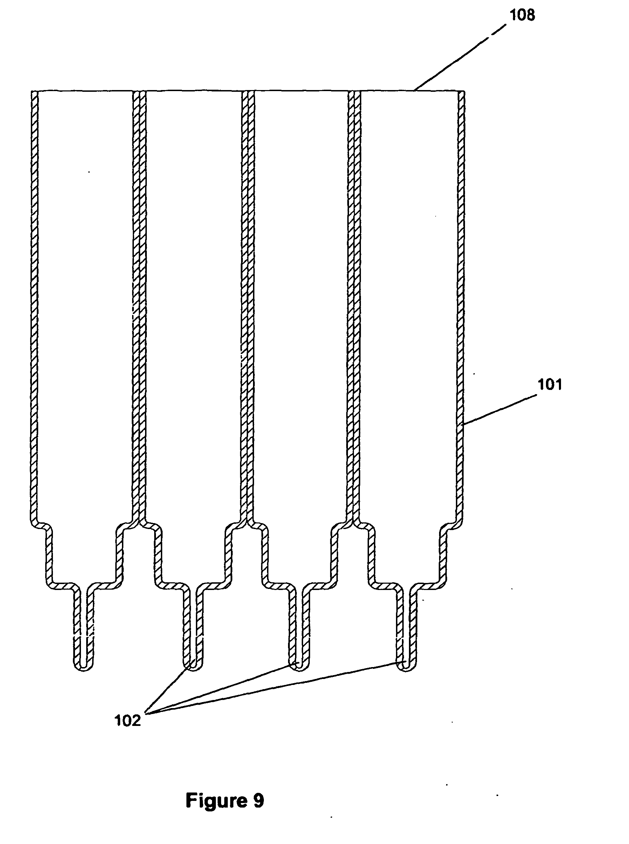

Located at one end of the central enclosure 4 and externally thereof are storage carousels 12 and 14 which can be accessed by the robot 2 through access doors 16 and 17 in a side wall common to the enclosure 4 and housing 5 of the carousels. The culture vessel assembly and culture aeration assembly which are generally indicated by the reference numerals 100 and 200 respectively, are shown most clearly in Figs 4 to 10 and will be described more fully below.

The carousels 12, 14 contain lab wear such as the cell culture vessel assembly 100 and culture aeration assembly 200, which are moved by the robot 2 about the system 1 at different stages during the process of producing protein.

The system 1 includes temperature controlled incubators 22 housed within an incubator enclosure 24. The incubator enclosure 24 is located at one end of the central enclosure 4, externally thereof and remote from the carousels 12 and 14. Access to the incubator enclosure 24 by the robot 2 is provided by an access door 25 which communicates between the central enclosure 4 and the incubator enclosure 24.

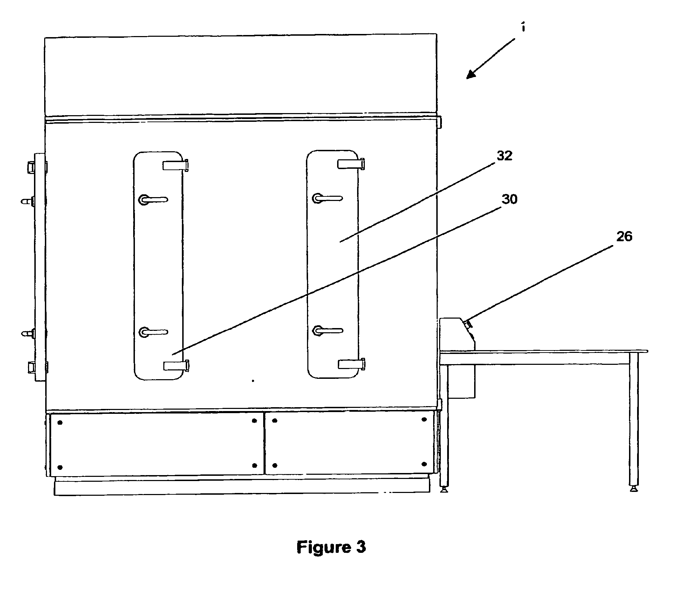

The cell culture system 1 further includes a computer control system 26 which controls the operation and working parameters of the system 1 from a remote user location. Access doors are provided on each enclosure 4, 5 and 24 to allow access for maintenance and replacement of equipment therein. Fig. 3 clearly shows doors 30 and 32 which provide manual access to the carousel housing 5.

Referring now to fig. 4, there is shown an embodiment of cell culture vessel assembly 100. The cell culture vessel assembly which is formed by injection moulding comprises a substantially rectangular shaped block 101 of 24 identical culture vessels or wells 102, having an open end 104 and a closed end 106. It will be apparent to the man skilled in the art that the cell culture vessel assembly may comprise any number of wells and the number will be tailored to suit the experiment to be carried out.

Each culture vessel or well 102 is generally in the shape of a rectangular cylinder having an open end 108 and a round closed end 110. The open and closed ends 108, 110 correspond to the open and closed ends 104, 106 of the culture vessel block 101. Each culture vessel or well 102 has a recessed portion 112 at the closed end 110. The recessed portion 112 generally extends from one side wall of the well 102 to midway towards an opposing side wall.

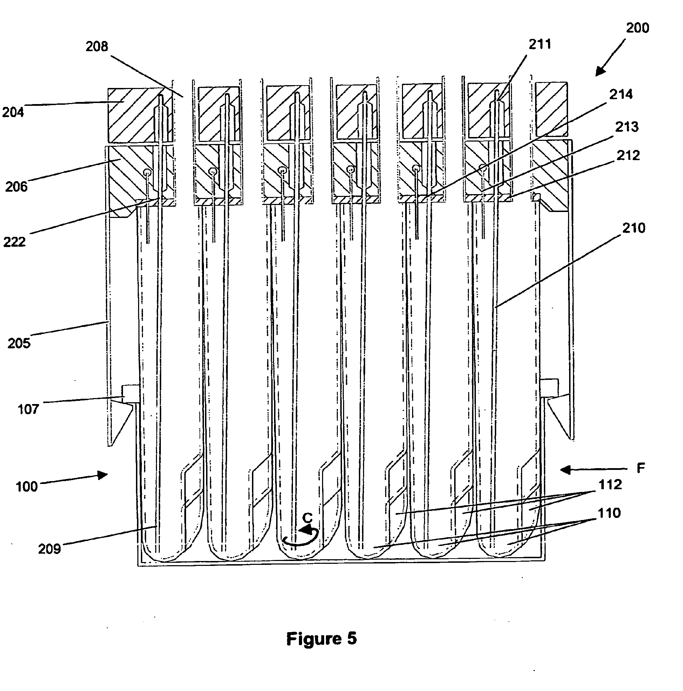

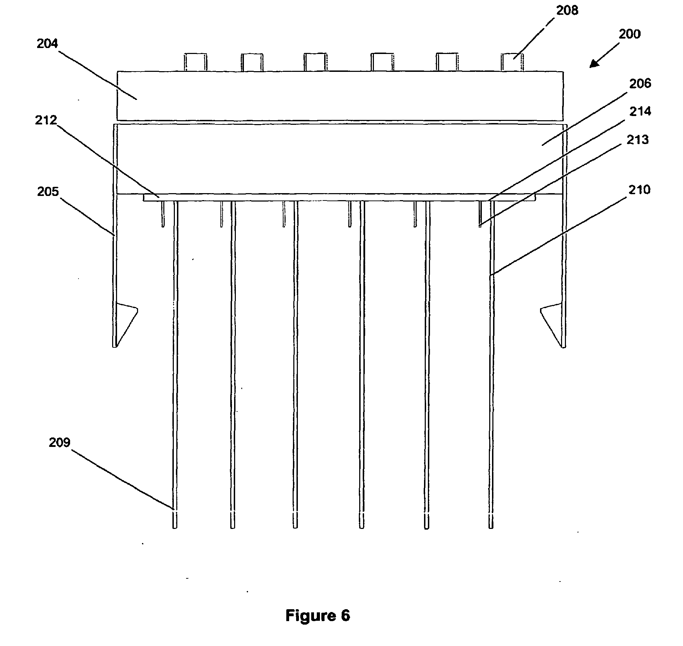

The culture vessel assembly 100 includes a lip 114 which extends about the periphery of the culture vessel block 101 at the open end 104 for engagement with the culture aeration assembly 200. The cell culture unit is shown most clearly in fig. 6, and in fig. 5 where it is shown attached to the culture vessel assembly.

The culture aeration assembly 200, comprises a lid 202 having an upper lid portion 204 and a lower lid portion 206. This is shown most clearly in fig. 5.

The upper and lower lid portions, or platens, 204, 206 have liquid media inlets 208 corresponding to each well 102, the lower lid portion 206 having stirring rods 210 attached thereto and extending perpendicularly from the lower lid 206, and air inlets 213 for introducing air into the wells 102. The stirring rods 210 are solid rod whisks and have a first end 209 which extends into the well 102 and a second end 211 which protrudes from the top surface of the lower lid 206. The culture aeration assembly 200 includes a rubber seal 212 which has perforations 214 to receive the stirring rods 210. The platens 204, 206 are relatively movable in a plane parallel to the plane of the platen. This relative movement results in the motion of the stirring rods within the wells 102. The upper lid portion 204 can additionally be covered by a closure portion (not shown) that provides a seal over the wells 102.

To assemble the culture vessel assembly 100 and culture aeration assembly 200, the seal 212 is positioned intermediate the open end 104 of the culture vessel block 101 and the lower lid 206 of the culture aeration assembly 200 so that the seal 212 is sandwiched between the culture vessel block 101 and the lower lid 206. The culture vessel block 101, lower lid 206 and seal 212 may be held together by conventional screws (not shown). Alternatively, the lower lid 206 may have securing arms 205 which extend to engage lugs 107 on the culture vessel block 101. This can be seen in fig 5.

When the seal 212 is placed over the open end 104 of the culture vessel block 101, the perforations 214 are in register with the open ends 108 of each well 102. The diameter of the perforations is such as to allow the stirring rods 210 to be pushed through the seal 212, the walls of each perforation 214 flexing to allow access of the stirring rods 210 into the wells 102, while maintaining the integrity of the seal 212 about the rods 210. When the lower lid 206 is engaged with the culture vessel block 101, each liquid inlet 208 is in register with a corresponding open end 108 of a culture vessel 101. Each liquid inlet 208 communicates with a corresponding vessel 101 through voids 220 in the seal 212 allowing the introduction of liquid medium into each well 102.

At stages in the protein production process and in particular during incubation, the upper lid 204 is engaged with the second end 211 of the stirring rods 210 which protrude from the top surface of the lower lid 206. This is shown most clearly in fig. 5. Cams (not shown) communicate with the upper lid 204 and rotate on an eccentric shaft which moves the upper lid 204 relative to the lower lid 206, seal 212 and culture vessel block 101. Movement of the upper lid 204 is transferred to the stirring rods 210 each of which move about a pivot point 222 located at the perforations 214 where the seal 212 contacts the stirring rods 210. The stirring rods 210 rotate within the culture. The pivoting movement of the stirring rod 210 is shown in fig. 5 in respect of a single well 102 where the arrow C represents a rotating motion about the pivot point 222. Fig. 5 shows that movement of the stirring rod 210 about the pivot point 222, minimises movement of the stirring rod 210 at the surface of the culture and maximises its movement near the closed end 110 of the well 102.

The effect of the pivoting movement of the stirring rod is twofold. Firstly, maximising movement of the stirring rod 210 at the closed end 110 of the well 102 helps re-suspend cells which may have settled on the bottom of the well 102. This is important so that all cells receive maximum aeration possible. Secondly, minimising movement of the stirring rod 210 at the surface of the culture minimises surface agitation and therefore minimises surface foaming of the culture. Surface foaming is undesirable as it reduces aeration of the culture and excessive foaming can lead to loss of culture and culture cells. When culturing E.coli it is preferable to incorporate as much air as possible into the culture. The addition of an anti-foaming agent to the culture improves the oxygenation of the cells as it allows oxygen-poor air to escape from the culture.

Furthermore, the increasing movement of the stirring rod 210 in the direction from the surface of the culture to the first end 209 of the stirring rod 210 creates a vortex in the culture which has the effect of drawing foam downwardly from the surface of the culture and dissipating it in the volume of the liquid culture. The vortex also draws air from the space above the surface of the culture into the volume of the liquid culture thus further improving aeration.

The air above the surface of the culture is replenished by introducing air into the well 102 through the air inlets 213 in the upper and lower lids 204, 206. Alternatively or in addition, the stirring rods 210 may have an air inlet and outlet (not shown) for introducing air directly into the culture. Air bubbles exiting the first end 209 of the stirring rods 210, for example, will be broken up into smaller bubbles by the movement of the stirring rods 210 thus further improving aeration of the culture.

## Claims

1. A system for aerating a plurality of cell samples contained in a plurality of wells, the system comprising:
a plurality of wells (102) arranged in a culture block (101);
at least one stirring rod (210) for each of the plurality of wells;
first and second platens (204, 206) arranged above the culture block and provided with connecting means for coupling each platen to each rod;
wherein relative motion between the first and second platens parallel to one another results in the rods stirring the contents of the wells.

2. The system according to claim 1, wherein the culture block is releasably connected to the platens.

3. The system according to claim 1 or claim 2, wherein the rods are retained by the platens when the culture block is released from the platens.

4. The system according to any of the preceding claims, wherein movement of the stirring rod increases along its length from adjacent the open end of the culture vessel towards the distal end of the stirring rod.

5. The system according to any of the preceding claims, wherein the rods are solid.

6. The system according to any of the preceding claims, wherein the rods are made from a Nickel Titanium alloy.

7. The system according to any of the preceding claims, wherein two rods enter each well, in use.

8. The system according to claim 7, wherein the two rods entering each well are of different lengths.

## Patentansprüche

1. System zum Belüften einer Mehrzahl Zellproben, die in einer Mehrzahl Behälter enthalten sind, wobei das System umfasst:
eine Mehrzahl Behälter (102), die in einem Block (101) von Kulturen angeordnet sind;
mindestens einen Rührstab (210) für jede der Mehrzahl Behälter;
erste und zweite Platten (204, 206), die über dem Block von Kulturen angeordnet und mit Verbindungsmitteln zum Koppeln jeder Platte mit jedem Stab versehen sind;
wobei die relative Bewegung zwischen den ersten und zweiten Platten parallel zu einander dazu führt, dass die Stäbe die Inhalte der Behälter rühren.

2. System nach Anspruch 1, wobei der Block von Kulturen lösbar mit den Platten verbunden ist.

3. System nach Anspruch 1 oder Anspruch 2, wobei die Stangen durch die Platten zurückgehalten werden, wenn der Block von Kulturen von den Platten gelöst wird.

4. System nach einem der vorstehenden Ansprüche, wobei die Bewegung des Rührstabs entlang seiner Länge von neben dem offenen Ende des Kulturgefäßes zu dem distalen Ende des Rührstabs hin zunimmt.

5. System nach einem der vorstehenden Ansprüche, wobei die Stäbe massiv sind.

6. System nach einem der vorstehenden Ansprüche, wobei die Stäbe aus einer Nickel-TitanLegierung hergestellt sind.

7. System nach einem der vorstehenden Ansprüche, wobei zwei Stäbe bei Verwendung in jeden Behälter eintreten.

8. System nach Anspruch 7, wobei die beiden Stäbe, die in jeden Behälter eintreten, unterschiedliche Längen aufweisen.

## Revendications

1. Système pour aérer une pluralité d'échantillons de cellules contenus dans une pluralité de cuvettes, le système comprenant :
une pluralité de cuvettes (102) disposées dans un bloc de culture (101) ;
au moins une tige d'agitateur (210) pour chacune de la pluralité de cuvettes ;
des première et seconde plaques (204, 206) disposées au-dessus du bloc de culture et munies de moyens de liaison pour coupler chaque plaque à chaque tige ;
dans lequel le mouvement relatif entre les première et seconde plaques parallèles l'une à l'autre entraîne que les tiges agitent le contenu des cuvettes.

2. Système selon la revendication 1, dans lequel le bloc de culture est lié de façon amovible aux plaques.

3. Système selon la revendication 1 ou 2, dans lequel les tiges sont maintenues par les plaques quand le bloc de culture est libéré des plaques.

4. Système selon l'une quelconque des revendications précédentes, dans lequel le déplacement de la tige d'agitateur augmente dans la direction de sa longueur depuis le voisinage de l'extrémité d'ouverture du récipient de culture vers l'extrémité distale de la tige d'agitateur.

5. Système selon l'une quelconque des revendications précédentes, dans lequel les tiges sont rigides.

6. Système selon l'une quelconque des revendications précédentes, dans lequel les tiges sont en alliage nickel titane.

7. Système selon l'une quelconque des revendications précédentes, dans lequel deux tiges entrent dans chaque cuvette en utilisation.

8. Système selon la revendication 7, dans lequel les deux tiges qui entrent dans chaque cuvette sont de longueurs différentes.
